(19) Europäisches Patentamt
European Patent Office
Office européen des brevets

(11) EP 1 023 399 B1

(12) EUROPEAN PATENT SPECIFICATION

(45) Date of publication and mention of the grant of the patent:
**02.07.2003 Bulletin 2003/27**

(21) Application number: **97945416.2**

(22) Date of filing: **30.09.1997**

(51) Int Cl.[7]: **C09B 47/30**, C07D 487/22, C09B 47/08 // (C07D487/22, 259:00, 209:00, 209:00, 209:00, 209:00)

(86) International application number:
**PCT/US97/17690**

(87) International publication number:
**WO 98/014521 (09.04.1998 Gazette 1998/14)**

(54) **NOVEL METHODS OF SYNTHESES OF PHTHALOCYANINE COMPOUNDS**

VERFAHREN ZUR SYNTHESE VON PHTHALOCYANINVERBINDUNGEN

TECHNIQUES DE SYNTHESE DE COMPOSES DE PHTALOCYANINE

(84) Designated Contracting States:
**AT CH DE FR GB IT LI**

(30) Priority: **01.10.1996 US 724347**

(43) Date of publication of application:
**02.08.2000 Bulletin 2000/31**

(73) Proprietor: **UNIVERSITY HOSPITALS OF CLEVELAND**
**Cleveland, Ohio 44106 (US)**

(72) Inventors:
- **Li. Ying-Syi**
  **Highland Heights, OH 44143 (US)**
- **KENNEY, Malcolm, E.**
  **Cleveland Heights, OH 44118 (US)**

(74) Representative: **Brewster, Andrea Ruth et al**
**MEWBURN ELLIS**
**York House**
**23 Kingsway**
**London WC2B 6HP (GB)**

(56) References cited:
**US-A- 5 403 928**
**US-A- 5 484 778**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 023 399 B1

Printed by Jouve, 75001 PARIS (FR)

## Description

### Background of the Invention

Conventional methods for synthesizing phthalocyanine pharmaceutical compounds, particularly HOSiPcOSi$(CH_3)_2(CH_2)_3N(CH_3)_2$, employ a blocking methyl group to prevent the addition of more than one aminosiloxy group on the central silicon of the phthalocyanine precursor. Once the aminosiloxy ligand has been added, this blocking group is displaced with the desired HO group through a photolysis step.

However, this method of synthesis has various disadvantages; photolysis requires unusual apparatus, is quite time consuming, and results in a relatively low yield, typically about 50 to 60% of the product. It would be desirable to have a method for synthesizing phthalocyanines, particularly HOSiPcOSi$(CH_3)_2(CH_2)_3N(CH_3)_2$ that does not involve a photolysis step, and provides an improved yield.

### Summary of the Invention

The present invention provides improved methods for synthesizing phthalocyanines, particularly HOSiPcOSi$(CH_3)_2(CH_2)_3N(CH_3)_2$, which do not involve photolysis, and which produce a purity of at least about 95%, and in the preferred embodiment of the first method of synthesizing Pc 4, provide a yield of greater than about 70% and typically greater than 80%. The first method involves a method for synthesizing a phthalocyanine compound comprising the following steps: providing a phthalocyanine precursor having a central silicon; adding a first aminosiloxy ligand to the central silicon of the phthalocyanine precursor; adding a second aminosiloxy ligand to the central silicon of the phthalocyanine precursor; displacing the second aminosiloxy ligand by an organic acid ligand, preferably a $Cl_3CCOO$ ligand; then displacing the $Cl_3CCOO$ ligand with an HO ligand.

The second method for making Pc 4 is a method for synthesizing a phthalocyanine compound comprising the following steps: providing a phthalocyanine precursor having a central silicon; providing a siloxy ligand with an iodo group; adding a first and a second siloxyiodo ligand to the central silicon of the phthalocyanine precursor; displacing the second siloxyiodo ligand by an organic acid ligand, preferably a $Cl_3CCOO$ ligand; then displacing the $Cl_3CCOO$ ligand with an HO ligand; and then displacing the iodo group with a dimethylamino group.

The invention also relates to methods for making two novel phthalocyanine compounds HOSiPcOSi$(CH_3)_2(CH_2)_3I$ and SiPc$[OSi(CH_3)_2(CH_2)_3I]_2$, and to the compounds themselves which are useful as dyes and in photodynamic therapy.

### Description of the Invention

The methods of the present invention are useful in making phthalocyanine compounds having the following general structure:

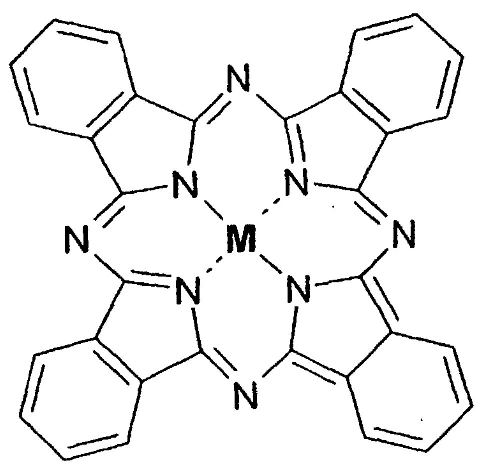

wherein: M is $(G)_aY[(OSi(CH_3)_2(CH_2)_bN_c(R')_d(R'')_e)_fX_g)_p$
Y is Si;

R' is selected from the group of H, C, $CH_2$, $CH_3$, $C_2H_5$, $C_4H_9$, $C_4H_8NH$, $C_4H_8N$, $C_4H_8NCH_3$,, $C_4H_8S$, $C_4H_8O$, $C_4H_8Se$, $CH_2CH_3$, $(CH_2)_3(CH_3)_2$, $OC(O)CH_3$, OC(O), $(CH_3)_2(CH_2)_{11}$, CS, CO, CSe, OH, $C_4H_8N(CH_2)_3CH_3$, $(CH_2)_3N(CH_3)_2$, C(O)$C_{27}H_{30}N_2O$, $(CH_2)_nN((CH)_o(CH_3))_2$, an alkyl group having from 1 to 12 carbon atoms;
R" is selected from the group of H, $SO_2CH_3$, $(CH_2)_2N(CH_3)_2$, $(CH_2)_{11}CH_3$, $C(S)NHC_6H_{11}O_5$, $(CH_2)_nN((CH)_o(CH_3))_2$, and an alkyl group having from 1 to 12 carbon atoms;
G is OH;
X is selected from the group of: I; F; Cl; or Br;

a = 0 or 1;
b = an integer from 2 to 12;
c = 0, 1;
d = 0, 1, 2, or 3;
e = 0, 1, or 2;
f = 1;
g = 0 or 1;
n = an integer from 1 to 12;
o = an integer from 1 to 11;
p = 1 where a is 1, or 2 where a is 0.

Preferably, M is $HOSiOSi(CH_3)_2(CH_2)_3N(CH_3)_2$, $HOSiOSi(CH_3)_2(CH_2)_3I$ or $Si[OSi(CH_3)_2(CH_2)_3I]_2$.
In the method for making Pc 4 of the prior art, the steps that are believed to occur are:

(1) $(CH_3O)_3Si(CH_2)_3N(CH_3)_2 + CH_3MgCl \rightarrow CH_3OSi(CH_3)_2(CH_2)_3N(CH_3)_2$

(2) $CH_3OSi(CH_3)_2(CH_2)_3N(CH_3)_2 + CH_3SiPcOH$

$\rightarrow CH_3SiPcOSi(CH_3)_2(CH_2)_3N(CH_3)_2$

(3) $CH_3SiPcOSi(CH_3)_2(CH_2)_3N(CH_3)_2 + O_2 + [H] \xrightarrow{\text{light}}$

$HOSiPcOSi(CH_3)_2(CH_2)_3N(CH_3)_2$

or

(4) $CH_3SiPcOSi(CH_3)_2(CH_2)_3N(CH_3)_2 + O_2 \xrightarrow{\text{light}}$

$HOSiPcOSi(CH_3)_2(CH_2)_3N(CH_3)_2$

where "H" represents a source of hydrogen. The byproducts in reactions (3) and (4) are different. The prior art synthesis uses a blocking methyl group to prevent the addition of more than one aminosiloxy group on the central silicon of the phthalocyanine precursor. Then, after the aminosiloxy ligand has been added, this blocking group is displaced with the desired HO group through a photolysis step.

## The First Method of Synthesizing Pc 4

In contrast to the prior art, the first new method of synthesis, avoids a photolysis step; an extra aminosiloxy ligand is added on the central silicon of the phthalocyanine precursor and then the sequential displacement of this extra ligand by an organic acid ligand; preferably a $Cl_3CCOO$ ligand and the desired HO ligand. The organic acid is a non-mineral acid, which has an acidity of $Cl_3CCOOH$, is soluble in the organic solvent used in the reaction, and includes but is not limited to $Cl_3CCOOH$ and $Cl_2CHCOOH$.
The first method involves a method for synthesizing a phthalocyanine compound comprising the following steps: providing a phthalocyanine precursor having a central silicon; adding a first aminosiloxy ligand to the central

silicon of the phthalocyanine precursor; adding a second aminosiloxy ligand to the central silicon of the phthalocyanine precursor; displacing the second aminosiloxy ligand by an organic acid ligand, preferably a $Cl_3CCOO$ ligand; then displacing the $Cl_3CCOO$ ligand with an HO ligand.

The steps in the preferred embodiment of the first synthesis, that are believed to occur are summarized as:

(5) $(CH_3O)_3Si(CH_2)_3N(CH_3)_2 + CH_3MgCl \rightarrow CH_3OSi(CH_3)_2(CH_2)_3N(CH_3)_2$

(6) $CH_3OSi(CH_3)_2(CH_2)_3N(CH_3)_2 + SiPc(OH)_2 \rightarrow$

$SiPc[OSi(CH_3)_2(CH_2)_3N(CH_3)_2]_2$

(7) $SiPc[OSi(CH_3)_2(CH_2)_3N(CH_3)_2]_2 + Cl_3CCOOH \rightarrow$

$Cl_3COOSiPcOSi(CH_3)_2(CH_2)_3N(CH_3)_2$

(8) $Cl_3COOSiPcOSi(CH_3)_2(CH_2)_3N(CH_3)_2 + H_2O \rightarrow$

$HOSiPcOSi(CH_3)_2(CH_2)_3N(CH_3)_2$

That is, the photolysis step is avoided; this results in a phthalocyanine product having an improved purity. The phthalocyanine produced according to the first improved method has a purity of about 98%; in contrast to the method of the prior art where the purity is about 95%. Further, the improved method provides a higher yield, that is, about 70% to 80%, rather than about 50% to 60%.

**The Second Synthesis of Pc 4**

The second synthesis of $HOSiPcOSi(CH_3)_2(CH_2)_3N(CH_3)_2$ uses a different approach from both the prior art and the first method. The second method for making Pc 4 is a method for synthesizing a phthalocyanine compound comprising the following steps: providing a phthalocyanine precursor having a central silicon; providing a siloxy ligand with an iodo group; adding a first and a second siloxyiodo ligand to the central silicon of the phthalocyanine precursor; displacing the second siloxyiodo ligand by an organic acid ligand, preferably a $Cl_3CCOO$ ligand; then displacing the $Cl_3CCOO$ ligand with an HO ligand; and then displacing the iodo group with a dimethylamino group.

The steps that probably occur in the preferred embodiment of the second method of synthesis of Pc 4 can be summarized as:

(9) $CH_3OSi(CH_3)_2(CH_2)_3Cl + NaI \rightarrow CH_3OSi(CH_3)_2(CH_2)_3I$

(10) $CH_3OSi(CH_3)_2(CH_2)_3I + SiPc(OH)_2 \rightarrow SiPc[OSi(CH_3)_2(CH_2)_3I]_2$

(11) $SiPc[OSi(CH_3)_2(CH_2)_3I]_2 + Cl_3CCOOH \rightarrow$

$Cl_3CCOOSiPcOSi(CH_3)_2(CH_2)_3I$

(12) $Cl_3CCOOSiPcOSi(CH_3)_2(CH_2)_3I + H_2O \rightarrow HOSiPcOSi(CH_3)_2(CH_2)_3I$

(13) $HOSiPcOSi(CH_3)_2(CH_2)_3I + HN(CH_3)_2 \rightarrow$

$HOSiPcOSi(CH_3)_2(CH_2)_3N(CH_3)_2$

In the second method for making Pc 4, a hydroxysiloxysilicon phthalocyanine in which the siloxy ligand terminates in an iodo group is first formed. Then, in the final reaction the iodo group is displaced with the desired dimethylamino group. Thus, in this approach the aminosiloxy ligand is built up stepwise on the phthalocyanine rather than being formed separately. The Pc 4 produced according to the second improved method has a purity of about 98%; in contrast to the methods of the prior art where the purity is about 95%.

This second synthesis is particularly useful because it offers a procedure for making Pc 4 labeled with $^{14}C$. The reagent $^{14}C$ labeled $NH(CH_3)_2$ is added in the last step of the method, preferably with unlabeled with $HN(CH_3)_2$. Suitable $^{14}C$ labeled $NH(CH_3)_2$ is commercially available from New England Nuclear Corporation, 549 Albany Street, Boston, MA 02118 and Amersham Life Science, Inc., 2636 South Clearbrook Drive, Arlington Heights, IL. The $^{14}C$ labeled Pc 4 is useful reagent for pharmacokinetic studies of Pc 4.

Compounds $HOSiPcOSi(CH_3)_2(CH_2)_3I$ and $SiPc[OSi(CH_3)_2(CH_2)_3I]_2$, which are made as intermediates in the method for making Pc 4, are also useful in photodynamic therapy, including therapies disclosed in U.S. Patent No. 5,166,197, issued November 24, 1992, to Kenney et. al.; U.S. Patent No. 5,484,778, issued January 16, 1996, to Kenney et. al.; and in "New Phthalocyanine Photosensitizers for Photodynamic Therapy", Oleinick, et. al. *Photochemistry and Photobiology,* (1993), Volume 57, pages 242-247. Compounds $HOSiPcOSi(CH_3)_2(CH_2)_3I$ and $SiPc[OSi(CH_3)_2(CH_2)_3I]_2$, are also useful as reagents, and for experimentation and research.

The following examples are intended to be illustrative only and not intended to in any way limit the scope of the invention.

Example 1 **Preferred Method for Synthesis of $HOSiPcOSi(CH_3)_2(CH_2)_3N(CH_3)_2$, Pc 4**

$HOSiPcOSi(CH_3)_2(CH_2)_3N(CH_3)_2$, Pc 4, was made by a synthesis in which $CH_3OSi(CH_3)_2(CH_2)_3N(CH_3)_2$, $SiPc[OSi(CH_3)_2(CH_2)_3N(CH_3)_2]_2$ (Pc 12), $Cl_3CCOOSiPcOSi(CH_3)_2(CH_2)_3N(CH_3)_2$, and finally Pc 4 were prepared in sequence.

Preparation of $CH_3OSi(CH_3)_2(CH_2)_3N(CH_3)_2$ $CH_3OSi(CH_3)_2(CH_2)_3N(CH_3)_2$ was prepared as described in U. S. Patent No. 5,166,197, issued November 24, 1992, to Kenney et al. and in "New Phthalocyanine Photosensitizers for Photodynamic Therapy", Oleinick, et. al. *Photochemistry and Photobiology*. 1993, volume 57, pages 242-247, at page 242.

**Preparation of $SiPc[OSi(CH_3)_2(CH_2)_3N(CH_3)_2]_2$;Pc 12**

A mixture of 980 mg, 5.59 mmol of $CH_3OSi(CH_3)_2(CH_2)_3N(CH_3)_2$ and a suspension of 400 mg, 0.697 mmol $SiPc(OH)_2$ and 400 mL pyridine that had been dried by distillation (about 20 mL of distillate), was slowly distilled for 3 hours (about 20 mL of distillate) and then filtered. The filtrate was evaporated to dryness with a rotary evaporator at about 40°C, and the solid was washed with 150 mL of a 2:3 ethanol-water solution, then dried at about 60 torr, about 70°C and weighed (552 mg). NMR analysis revealed (300 MHz, $CDCl_3$): delta 9.61 (m, 1,4-Pc H), 8.31 (m, 2,3-Pc H), 1.60 (s, $NCH_3$), 0.82 (m, gamma-$CH_2$), -1.12 (m, beta-$CH_2$), -2.30 (m, alpha-$CH_2$), -2.91 (s, $SiCH_3$). The resulting $SiPc[OSi(CH_3)_2(CH_2)_3N(CH_3)_2]_2$ which is blue, is soluble in dimethylformamide and $CH_2Cl_2$, and is insoluble in hexanes and water.

Preparation of **$Cl_3CCOOSiPcOSi(CH_3)_2(CH_2)_3N(CH_3)_2$**

A solution of 552 mg of $SiPc[OSi(CH_3)_2(CH_2)_3N(CH_3)_2]_2$ also referred to herein as "Pc 12", 590 mg, 3.61 mmol trichloroacetic acid, and 90 mL $CH_2Cl_2$, was stirred at room temperature for 5 hours. The resultant was mixed with 90 mL pyridine and then with 90 mL $H_2O$, and the mixture formed was separated. The aqueous layer was washed with 100 mL $CH_2Cl_2$, the washings were filtered, and the residue was washed with 200 mL $CH_2Cl_2$The organic layer was filtered, and the . filtrate and $CH_2Cl_2$ washings were combined and evaporated to dryness with a rotary evaporator at about 40°C. The solid was dried at about 60 torr, and at about 60°C to provide 647 mg of the blue-green compound, $Cl_3CCOOSiPcOSi-(CH_3)_2(CH_2)_3N(CH_3)_2$. NMR analysis revealed (300 MHz, $CDCl_3$) : delta 9.68 (m, 1,4-Pc H), 8.19 (m, 2,3-Pc H), 1.61 (s, $NCH_3$), 0.85 (m, gamma-$CH_2$), -1.03 (m, beta-$CH_2$), -2.16 (m, alpha-$CH_2$), -2.78 (s, $SiCH_3$).

**Preparation of $HOSiPcOSi(CH_3)_2(CH_2)_3N(CH_3)_2$, Pc 4**

A solution of 647 mg $Cl_3CCOOSiPcOSi(CH_3)_2(CH_2)_3N(CH_3)_2$ and 20 mL $CH_2Cl_2$ was passed down a 3 x 24 cm chromatography column composed of $Al_2O_3$ V. The loaded column was then eluted with a solution of 1.5 parts $CH_2Cl_2$-ethyl acetate, and the eluate was evaporated to dryness with a rotary evaporator at about 40°C. The solid was extracted into 20 mL $CH_2Cl_2$, recovered by evaporating the extract with a rotary evaporator, dissolved in 3 ml of $CH_2Cl_2$, and precipitated with 12 mL pentane. The precipitate was recovered by filtration, washed with a 15 mL of a 1:4 $CH_2Cl_2$-

pentane solution and 20 mL pentane, dried at about 60 torr, 60°C, and weighed (416 mg, 0.580 mmol, 83% based on $SiPc(OH)_2$ used). UV-vis (dimethylformamide) $lambda_{max}$ (nm); epsilon ($M^{-1}cm^{-1}$): 668; 230,000. NMR analysis revealed (300 MHz, 8.4 mM, $CDCl_3$): delta 9.11 (m, 1,4-Pc H), 8.18 (m, 2,3-Pc H), 1.35 (s, $NCH_3$), 0.60 (m, gamma-$CH_2$), -1.36 (m, beta-$CH_2$), -2.49 (m, alpha-$CH_2$), -2.66 (s, SiOH), -3.10 (s, $SiCH_3$).

**Example 2 Second Method for Synthesis of Pc 4.**

Pc 4 was also made by a synthesis in which $CH_3OSi(CH_3)_2(CH_2)_3I$, $SiPc[OSi(CH_3)_2(CH_2)_3I]_2$ also referred to herein as "Pc 59", $HOSiPcOSi(CH_3)_2(CH_2)_3I$ also referred to herein as "Pc 58", and finally Pc 4 were prepared in sequence.

**Preparation of $CH_3OSi(CH_3)_2(CH_2)_3I$**

A mixture of 10.6 g, 0.0700 mol $CH_3OSi(CH_3)_2(CH_2)_3Cl$, 20.8 g, 0.105 mol NaI and 100 mL acetone was refluxed for 2 days and filtered. The solid was washed with 50 mL acetone, and the washings and filtrate were combined and concentrated with a rotary evaporator at about 30°C. The concentrate was mixed with 50 mL ether, and the resulting suspension was filtered. The solid was washed with 100 ml ether, and the washings and the filtrate were combined and concentrated with a rotary evaporator at about 30°C. The concentrate weighed 14.9 g. NMR analysis at 200 MHz, in $CDCl_3$ revealed: delta 3.40 (s, $CH_3O$), 3.17 (m, gamma-$CH_2$), 1.84 (m, beta-$CH_2$), 0.67 (m, alpha-$CH_2$), 0.08 (s, $SiCH_3$). The $CH_3OSi(CH_3)_2(CH_2)_3I$ is a yellow, mobile liquid.

**Preparation of $SiPc[OSi(CH_3)_2(CH_2)_3I]_2$**

A mixture of 1.80 g $CH_3OSi(CH_3)_2(CH_2)_3I$, 401 mg, 0.698 mmol $SiPc(OH)_2$ and 300 mL xylene was slowly distilled for 6 hours to produce about 35 mL of distillate, and the mixture was evaporated to dryness with a rotary evaporator at about 50°C. The solid was washed with 75 mL ethanol and 70 mL of a 1:1 ethanol-water solution, then dried at about 60 torr, 60°C, and weighed (567 mg, 0.537 mmol, 77 %). NMR analysis at 300 MHz, in $CDCl_3$ revealed: delta 9.65 (m, 1,4-Pc H), 8.34 (m, 2,3-Pc H), 1.75 (m, gamma-$CH_2$), -0.84 (m, beta-$CH_2$), - 2.21 (m, alpha-$CH_2$), -2.87 (s, $SiCH_3$). The $SiPc[OSi(CH_3)_2(CH_2)_3I]_2$, which is blue, is soluble in dimethylformamide and $CH_2Cl_2$ and is insoluble in hexanes and water.

**Preparation of $HOSiPcOSi(CH_3)_2(CH_2)_3I$**

A solution of 514 mg, 0.488 mmol unchromatographed $SiPc[OSi(CH_3)_2(CH_2)_3I]_2$, 1.64 g, 10.0 mmol trichloroacetic acid and 250 mL $CH_2Cl_2$ was stirred at room temperature for 5 hours, then mixed with 100 mL pyridine and then with 100 mL $H_2O$. The aqueous layer was washed with 60 mL $CH_2Cl_2$, the washings were filtered, and the residue was washed with 100 mL $CH_2Cl_2$. The organic layer was filtered and the filtrate and $CH_2Cl_2$ washings were combined and evaporated to dryness with a rotary evaporator (about 40°C). The solid was chromatographed on an $Al_2O_3$ V substrate, with $CH_2Cl_2$-ethyl acetate, in a 1:1 ratio, dissolved in 4 mL $CH_2Cl_2$, precipitated with 6 mL pentane, recovered by filtration, washed with 20 mL pentane and a 1:1, 20 mL ethanol-water solution, dried at about 60 torr, about 60°C and weighed (188 mg, 0.235 mmol, 48%). NMR analysis revealed (300 MHz, 7.0 mM, $CDCl_3$): delta 9.22 (m, 1,4-Pc H), 8.22 (m, 2,3-Pc H), 1.59 (m, gamma-$CH_2$), -1.01 (m, beta-$CH_2$), -2.36 (m, alpha-$CH_2$), -3.04 (s, $SiCH_3$), -3.22 (s, SiOH). The $HOSiPcOSi(CH_3)_2(CH_2)_3I$, which is blue, is soluble in dimethylformamide and $CH_2Cl_2$ and is insoluble in hexanes and water.

**Preparation of $HOSiPcOSi(CH_3)_2(CH_2)_3N(CH_3)_2$, Pc 4, the Final Stage**

A stirred suspension of 30.9 mg, 0.0386 mmol $HOSiPcOSi(CH_3)_2(CH_2)_3I$, 5.2 mg, 0.038 mmol $K_2CO_3$, 0.5 mL (1 mmol) of a 2M solution of $HN(CH_3)_2$ in tetrahydrofuran and 9.5 mL tetrahydrofuran in a pressure tube (15 mL) was warmed at about 60°C for 48 hours and evaporated to dryness with a rotary evaporator at about 30°C. The solid was extracted into 20 mL $CH_2Cl_2$, recovered by evaporating the extract with a rotary evaporator (room temperature), dissolved in 1 mL $CH_2Cl_2$, precipitated with 4 mL pentane, recovered by filtration, washed with 15 mL of a 1:4 solution of $CH_2Cl_2$ and pentane, 10 mL pentane and 10 mL of a 1:4 ethanol-water solution, dried at about 60 torr, at about 60°C and weighed (22.1 mg, 0.0308 mmol, 80 %, 30 % based on $SiPc(OH)_2$ used).

Example 2a

The procedure of Example 2 was followed, except in the final stage a smaller ratio of $HN(CH_3)_2$ to HOSiPcOSi

$(CH_3)_2(CH_2)_3I$ was employed. Specifically, a stirred suspension of 40.5 mg, 0.0506 mmol $HOSiPcOSi(CH_3)_2(CH_2)_3I$, PC 58, 2.4 mg, 0.017 mmol $K_2CO_3$, 0.2 mL (0.4 mmol) of a 2M solution of $HN(CH_3)_2$ in tetrahydrofuran and 4.8 mL tetrahydrofuran in a 5 mL heavy-wall reaction vial was warmed at about 60°C for 24 hours, and evaporated to dryness with a rotary evaporator at about 30°C. The solid was extracted into 20 mL $CH_2Cl_2$, recovered by evaporating the extract to dryness with a rotary evaporator at room temperature, dissolved in 1 mL $CH_2Cl_2$, precipitated with 4 mL pentane, recovered by filtration, washed with 15 mL of a 1:4 $CH_2Cl_2$-pentane solution, 10 mL pentane and 10 mL of a 1:4 ethanol-water solution, dried at about 60 torr, at about 60°C, chromatographed on a $Al_2O_3$ III substrate using ethyl acetate-methanol, in a ratio of 10:1, washed with 15 mL pentane and 10 mL of a 1:4 ethanol-water solution, dried at about 60 torr, 60°C and weighed (17.5 mg, 0.0243 mmol, 48%, 18% based on $SiPc(OH)_2$ used).

Example 2b

The synthesis of Example 2 is followed, except that a metallic derivative of $HN(CH_3)_2$, preferably a group I metal derivative, including, for example $LiN(CH_3)_2$ and $NaN(CH_3)_2$, is substituted for $HN(CH_3)_2$. Since the halosiloxy phthalocyanine and the amine may be used in an approximately 1:1 ratio, and the reaction thus is more efficient.

Example 2c

The procedure of Example 2 is followed except that a mixture of $^{14}C$ labeled $NH(CH_3)_2$ and $HN(CH_3)_2$, is added in the last reaction with to provide Pc 4 labeled with $^{14}C$.

Although one embodiment of this invention has been shown and described, various adaptations and modifications can be made without departing from the scope of the invention as defined in the appended claims.

## Claims

**1.** A method for synthesizing a phthalocyanine compound comprising the following steps:

a. providing a phthalocyanine precursor having a central silicon;
b. adding two aminosiloxy ligands to the central silicon of the phthalocyanine precursor;
c. displacing one of the aminosiloxy ligands by an organic acid ligand;
d. then displacing the organic acid ligand with an HO ligand.

**2.** A method according to claim 1, wherein
the phthalocyanine compound is $HOSiPcOSi(CH_3)_2(CH_2)_3N(CH_3)_2$,
the phthalocyanine precursor is $SiPc(OH)_2$, and
the amino siloxy ligand is $CH_3OSi(CH_3)_2(CH_2)_3N(CH_3)_2$.

**3.** A method according to claim 1 or 2, wherein the organic acid ligand is $Cl_3CCOO$.

**4.** A method according to any one of claims 1 to 3, wherein the synthesis is accomplished without photolysis.

**5.** A method for synthesizing a phthalocyanine compound comprising the following steps:

a. providing a phthalocyanine precursor having a central silicon;
b. providing a siloxy ligand with an iodo group;
c. adding a first and a second siloxyiodo ligand to the central silicon of the phthalocyanine precursor.

**6.** A method according to claim 5, wherein the phthalocyanine produced from step c is $SiPc[OSi(CH_3)_2(CH_2)_3I]_2$.

**7.** A method according to claim 5 or 6, further comprising the steps of:

d. displacing one of the two siloxyiodo ligands by an organic acid ligand;
e. then displacing the organic acid ligand with an HO ligand.

**8.** A method according to claim 7, wherein the organic acid ligand is $Cl_3CCOO$.

**9.** A method according to claim 10, wherein the phthalocyanine compound is $HOSiPcOSi(CH_3)_2(CH_2)_3N(CH_3)_2$.

**10.** A method according to any one of claims 7 or 8, further comprising the step of:

f. then displacing the iodo group with a dimethylamino group.

**11.** A method according to claim 10, wherein the dimethylamino group is $^{14}C$ labeled.

**12.** A method according to claim 10, wherein the dimethylamino group is provided by a group I metal dimethylamine.

**13.** The phthalocyanine compound $HOSiPcOSi(CH_3)_2(CH_2)_3I$.

**14.** The phthalocyanine compound $SiPc[OSi(CH_3)_2(CH_2)_3I]_2$.

**Patentansprüche**

**1.** Verfahren zum Synthetisieren einer Phthalocyanin-Verbindung, die folgenden Schritte umfassend:

a) Bereitstellen eines Phthalocyanin-Vorläufers mit einem zentralen Silizium;
b) Hinzufügen zweier Aminosiloxy-Liganden zum zentralen Silizium des Phthalocyanin-Vorläufers;
c) Verdrängen eines der Aminosiloxy-Liganden durch einen organischen Säure-Liganden;
d) anschließendes Verdrängen des organischen Säure-Liganden mit einem HO-Liganden.

**2.** Verfahren nach Anspruch 1, worin:

die Phthalocyanin-Verbindung $HOSiPcOSi(CH_3)_2(CH_2)_3N(CH_3)_2$ ist,
der Phthalocyanin-Vorläufer $SiPc(OH)_2$ ist und
der Aminosiloxy-Ligand $CH_3OSi(CH_3)_2(CH_2)_3N(CH_3)_2$ ist.

**3.** Verfahren nach Anspruch 1 oder 2, worin der organische Säure-Ligand $Cl_3CCOO$ ist.

**4.** Verfahren nach einem der Ansprüche 1 bis 3, worin die Synthese ohne Photolyse durchgeführt wird.

**5.** Verfahren zum Synthetisieren einer Phthalocyanin-Verbindung, die folgenden Schritte umfassend:

a) Bereitstellen eines Phthalocyanin-Vorläufers mit einem zentralen Silizium;
b) Bereitstellen eines Siloxy-Liganden mit einer Iod-Gruppe;
c) Hinzufügen eines ersten und eines zweiten Siloxyiod-Liganden zum zentralen Silizium des Phthalocyanin-Vorläufers.

**6.** Verfahren nach Anspruch 5, worin das in Schritt c) hergestellte Phthalocyanin $SiPc[OSi(CH_3)_2(CH_2)_3I]_2$ ist.

**7.** Verfahren nach Anspruch 5 oder 6, das weiters folgende Schritte umfasst:

d) Verdrängen eines der beiden Siloxyiod-Liganden durch einen organischen Säure-Liganden;
e) anschließendes Verdrängen des organischen Säure-Liganden mit einem HO-Liganden.

**8.** Verfahren nach Anspruch 7, worin der organische Säure-Ligand $Cl_3CCOO$ ist.

**9.** Verfahren nach Anspruch 10, worin die Phthalocyanin-Verbindung $HOSiPcOSi\text{-}(CH_3)_2(CH_2)_3N(CH_3)_2$ ist.

**10.** Verfahren nach einem der Ansprüche 7 oder 8, weiters den folgenden Schritt umfassend:

f) anschließendes Verdrängen der Iod-Gruppe mit einer Dimethylaminogruppe.

**11.** Verfahren nach Anspruch 10, worin die Dimethylaminogruppe $^{14}C$-markiert ist.

**12.** Verfahren nach Anspruch 10, worin die Dimethylaminogruppe durch ein Gruppe I-Metall-Dimethylamin bereitgestellt wird.

**13.** Die Phthalocyanin-Verbindung $HOSiPcOSi(CH_3)_2(CH_2)_3I$.

**14.** Die Phthalocyanin-Verbindung $SiPc[OSi(CH_3)_2(CH_2)_3I]_2$.

## Revendications

**1.** Méthode pour la synthèse d'un composé de phtalocyanine comprenant les étapes suivantes :

a. prévoir un précurseur de phtalocyanine ayant un silicium central ;
b. ajouter deux ligands aminosiloxy au silicium central du précurseur de phtalocyanine ;
c. déplacer l'un des ligands aminosiloxy par un ligand d'acide organique ;
d. puis déplacer le ligand d'acide organique par un ligand HO.

**2.** Méthode selon la revendication 1, où
le composé de phtalocyanine est $HOSiPcOSi(CH_3)_2(CH_2)_3N(CH_3)_2$,
le précurseur de phtalocyanine est $SiPc(OH)_2$, et
le ligand aminosiloxy est $CH_3Osi(CH_3)_2(CH_2)_3N(CH_3)_2$.

**3.** Méthode selon la revendication 1 ou 2, où le ligand d'acide organique est $Cl_3CCOO$.

**4.** Méthode selon l'une quelconque des revendications 1 à 3, où la synthèse est accomplie sans photolyse.

**5.** Méthode pour la synthèse d'un composé de phtalocyanine comprenant les étapes suivantes :

a. prévoir un précurseur de phtalocyanine ayant un silicium central ;
b. prévoir un ligand siloxy avec un groupe iodo ;
c. ajouter un premier et un second ligand siloxyiodo au silicium central du précurseur de phtalocyanine.

**6.** Méthode selon la revendication 5, où la phtalocyanine produite à partir de l'étape c est $SiPc[Osi(CH_3)_2(CH_2)_3I]_2$.

**7.** Méthode selon la revendication 5 ou 6, comprenant de plus les étapes de :

d. déplacer l'un des deux ligands siloxyiodo par un ligand d'acide organique ;
e. puis déplacer le ligand d'acide organique par un ligand HO.

**8.** Méthode selon la revendication 7, où le ligand d'acide organique est $Cl_3CCOO$.

**9.** Méthode selon la revendication 10, où le composé de phtalocyanine est $HOSiPcOSi(CH_3)_2(CH_2)_3N(CH_3)_2$.

**10.** Méthode selon l'une quelconque des revendications 7 ou 8, comprenant de plus l'étape de :

f. déplacer alors le groupe iodo par un groupe diméthylamino.

**11.** Méthode selon la revendication 10, où le groupe diméthylamino est marqué $^{14}C$.

**12.** Méthode selon la revendication 10, où le groupe diméthylamino est formé d'une diméthylamine d'un métal du groupe I.

**13.** Composé de phtalocyanine $HOSiPcOSi(CH_3)_2(CH_2)_3I$.

**14.** Composé de phtalocyanine $SiPc[Osi(CH_3)_2(CH_2)_3I]_2$.